# EUROPEAN PATENT SPECIFICATION

(11) **EP 3 185 892 B1**
(45) Date of publication and mention of the grant of the patent: **12.02.2020**
(21) Application number: 15756141.6
(22) Date of filing: 24.08.2015
(51) Int. Cl.: A61K 38/55, A61K 38/57

(54) **USE OF ELAFIN FOR DISORDERS ASSOCIATED WITH ELASTASE INDEPENDENT INCREASE IN TROPONIN**
VERWENDUNG VON ELAFIN FÜR ERKRANKUNGEN IM ZUSAMMENHANG MIT ELASTASEUNABHÄNGIGEM ANSTIEG VON TROPONIN
UTILISATION DE L'ÉLAFINE POUR LES TROUBLES ASSOCIÉS À L'AUGMENTATION DE LA TROPONINE INDÉPENDANTE DE L'ÉLASTASE

(30) Priority: 26.08.2014 DE 102014216985
(43) Date of publication of application: 05.07.2017
(73) Proprietor: Proteo Biotech AG, 24106 Kiel (DE)
(72) Inventor: BARGMANN, Birge, 24105 Kiel (DE); WIEDOW, Oliver, 24105 Kiel (DE); HENRIKSEN, Peter, Edinburgh EH16 5NH (GB)
(74) Representative: Hoffmann Eitle
(86) International application number: PCT/EP2015/069341
(87) International publication number: WO 2016/030323

(56) References cited:
- WO-A1-2011/107505
- WO-A2-99/43308
- WO-A2-03/090682
- LEE SHAW ET AL: "Therapeutic potential of human elafin: Figure 1", BIOCHEMICAL SOCIETY TRANSACTIONS, vol. 2, no. 5, 1 October 2011 (2011-10-01) , pages 749-1454, XP055219347, GB ISSN: 0300-5127, DOI: 10.1093/ndt/gfq041
- ALAM SHIRJEL R ET AL: "Role of the endogenous elastase inhibitor, elafin, in cardiovascular injury From epithelium to endothelium", BIOCHEMICAL PHARMACOLOGY, vol. 83, no. 6, 2012, pages 695-704, XP028890393, ISSN: 0006-2952, DOI: 10.1016/J.BCP.2011.11.003
- Anonym: "Elafin granted second FDA orphan drug designation", Prous integrity , 22 March 2013 (2013-03-22), XP002745733, Retrieved from the Internet: URL:https://integrity.thomson-pharma.com/i ntegrity/xmlxsl/pk_ref_list.xml_show_ficha _ref?p_ref_id=1961448 [retrieved on 2015-10-09]
- S. H.E. ZAIDI: "Overexpression of the Serine Elastase Inhibitor Elafin Protects Transgenic Mice From Hypoxic Pulmonary Hypertension", CIRCULATION, vol. 105, no. 4, 29 January 2002 (2002-01-29), pages 516-521, XP0055219355, US ISSN: 0009-7322, DOI: 10.1161/hc0402.102866
- O'BLENES S B ET AL: "Gene transfer of the serine elastase inhibitor elafin protects against vein graft degeneration", CIRCULATION, LIPPINCOTT WILLIAMS & WILKINS, US, vol. 102, no. 19, 7 November 2000 (2000-11-07), pages III.289-III.295, XP002350040, ISSN: 0009-7322
- Kunio Ohta ET AL: "Elafin-overexpressing mice have improved cardiac function after myocardial infarction", American Journal of Physiology - Heart and Circulatory Physiology, 1 July 2004 (2004-07-01), pages 286-292, XP055219351, United States DOI: 10.1152/ajpheart.00479.2002 Retrieved from the Internet: URL:http://ajpheart.physiology.org/content /287/1/H286.full.pdf
- COWAN B ET AL: "Elafin, a serine elastase inhibitor, attenuates post-cardiac transplant coronary arteriopathy and reduces myocardial necrosis in rabbits afer heterotopic cardiac transplantation", JOURNAL OF CLINICAL INVESTIGATION, AMERICAN SOCIETY FOR CLINICAL INVESTIGATION, US, vol. 97, no. 11, 1 June 1996 (1996-06-01), pages 2452-2468, XP009172925, ISSN: 0021-9738
- A. W. BAROLET ET AL: "Arterial Elastase Activity After Balloon Angioplasty and Effects of Elafin, an Elastase Inhibitor", ARTERIOSCLEROSIS, THROMBOSIS, AND VASCULAR BIOLOGY., vol. 21, no. 8, 1 August 2001 (2001-08-01) , pages 1269-1274, XP055219753, US ISSN: 1079-5642, DOI: 10.1161/hq0801.093589

### SEQUENCE LISTING

<110> Proteo Biotech AG
<120> Novel uses of Elafin
<130> P 67904, HE 183 576
<160> 4
<170> PatentIn version 3.4
<210> 1
   <211> 57
   <212> PRT
   <213> Homo sapiens
<400> 1
   Ala Gln Glu Pro Val Lys Gly Pro Val Ser Thr Lys Pro Gly Ser Cys
   1               5                   10                  15
   Pro Ile Ile Leu Ile Arg Cys Ala Met Leu Asn Pro Pro Asn Arg Cys
               20                  25                  30
   Leu Lys Asp Thr Asp Cys Pro Gly Ile Lys Lys Cys Cys Glu Gly Ser
           35                  40                  45
   Cys Gly Met Ala Cys Phe Val Pro Gln
       50                  55
<210> 2
   <211> 117
   <212> PRT
   <213> Homo sapiens
<400> 2
   Met Arg Ala Ser Ser Phe Leu Ile Val Val Val Phe Leu Ile Ala Gly
   1               5                   10                  15
   Thr Leu Val Leu Glu Ala Ala Val Thr Gly Val Pro Val Lys Gly Gln
               20                  25                  30
   Asp Thr Val Lys Gly Arg Val Pro Phe Asn Gly Gln Asp Pro Val Lys
           35                  40                  45
   Gly Gln Val Ser Val Lys Gly Gln Asp Lys Val Lys Ala Gln Glu Pro
       50                  55                  60
   Val Lys Gly Pro Val Ser Thr Lys Pro Gly Ser Cys Pro Ile Ile Leu
   65                  70                  75                  80
   Ile Arg Cys Ala Met Leu Asn Pro Pro Asn Arg Cys Leu Lys Asp Thr
                   85                  90                  95
   Asp Cys Pro Gly Ile Lys Lys Cys Cys Glu Gly Ser Cys Gly Met Ala
               100                 105                 110
   Cys Phe Val Pro Gln
           115
<210> 3
   <211> 623
   <212> DNA
   <213> Homo sapiens
<400> 3
   aggccaagct ggactgcata aagattggta tggccttagc tcttagccaa acaccttcct      60
   gacaccatga gggccagcag cttcttgatc gtggtggtgt tcctcatcgc tgggacgctg     120
   gttctagagg cagctgtcac gggagttcct gttaaaggtc aagacactgt caaaggccgt     180
   gttccattca atggacaaga tcccgttaaa ggacaagttt cagttaaagg tcaagataaa     240
   gtcaaagcgc aagagccagt caaaggtcca gtctccacta agcctggctc ctgccccatt     300
   atcttgatcc ggtgcgccat gttgaatccc cctaaccgct gcttgaaaga tactgactgc     360
   ccaggaatca agaagtgctg tgaaggctct tgcgggatgg cctgtttcgt tccccagtga     420
   gagggagccg gtccttgctg cacctgtgcc gtccccagag ctacaggccc catctggtcc     480
   taagtccctg ctgcccttcc ccttcccaca ctgtccattc ttcctcccat tcaggatgcc     540
   cacggctgga gctgcctctc tcatccactt tccaataaag agttccttct gctccaaaaa     600
   aaaaaaaaaa aaaaaaaaaa aaa                                             623
<210> 4
   <211> 6
   <212> PRT
   <213> Künstliche Sequenz
<220>
   <223> Repeat sequence
<220>
   <221> PROPEP
   <222> (4)..(4)
   <223> Xaa can be any naturally occurring amino acid
<400> 4
   Gly Gln Asp Xaa Val Lys
   1               5

## Description

### FIELD AND BACKGROUND OF THE INVENTION

The present invention relates to the use of elafin for the treatment and/or prevention of diseases or disorders associated with an increase in troponin levels, which are non elastase dependent. The present invention in a preferred embodiment relates to a method and composition, using elafin, for protecting the heart muscle or other muscles from damage induced by abnormal blood flow and/or inflammation, which may result from, for example, a heart infarction. The present invention additionally or concomitantly relates to the use of elafin for the treatment and/or prevention of disorders or diseases which are associated with a rise in the level of troponin I and/or T.

Elafin is a recombinant human protein, known to act as a reversible tight binding inhibitor of elastase and the closely related serine protease proteinase 3.

Elafin can be taken up from the extracellular medium by monocytes [Butler et al., 2006] and vascular endothelial cells [Nickel et al., 2013]. The full citations are indicated at the end of the Background Section.

Elafin can interfere with intracellular signaling events. In the monocytic cell line U937 elafin inhibited the LPS-induced activation of the transcription factors AP-1 and NF-κB via an effect on the ubiquitin-proteasome pathway, with no inhibition of peptidase activities associated with the 20 S proteasome. This led to inhibition of the production of the potent proinflammatory factor macrophage chemokine MCP-1 (macrophage chemoattractant protein-1; CCL2) [Butler et al., 2006].

Over-expression of elafin by gene transfer suppressed an inflammatory response in cultured human umbilical vein endothelial cells (HUVEC). The release of IL-8 by HUVEC in response to a challenge with oxidized low density lipoprotein, LPS and TNFα was reduced by elafin overexpression. In addition, elafin overexpression in macrophages attenuated the LPS-stimulated release of the proinflammatory cytokine TNFα. In both cell types these effects were associated with reduced activation of the inflammatory transcription factor NF-κB, through up-regulation of IκBα [Henriksen et al., 2004].

In spite of the plethora of scientific research being done on elafin, the one commonly known and established action is its effect on elastase (and the serine protease proteinase 3 which has similar active roles in initiating and sustaining an inflammatory response). There is no confirmed indication of an effect of elafin on any particular further compound of interest in the treatment or prevention of disorders. In particular, the only effects of elafin known regarding the treatment of disorders or diseases encountered in the heart and/or during surgery, in particular of the heart, are those, where it is known that elastase is the causing agent of such disorders or diseases. Indeed, this is true not only for diseases of the heart muscle, but also for all disorders involving further muscles in the human/animal body. Additionally, there is no known connection between the release of troponins, in particular cardiac troponin I or cardiac troponin T or skeletal troponin T, and elafin.

Up to now, elafin has in particular been known to be a highly specific, potent and reversible inhibitor for neutrophil-derived elastase and proteinase 3. Its activity was shown to be largely restricted to epithelial tissues.

Accordingly, elafin has been proposed as a suitable agent for the treatment or prevention of e.g. inflammatory diseases which have been shown to be the result of abundant amounts of elastase, e.g. SIRS (systemic inflammation response syndrome) or MODS (multiple-organ dysfunction syndrome).

There has been no report on an association of elafin and troponin-levels.

The heart muscle is composed of millions of contractile cardiomyocytes that are responsible for the pump function of the heart.

Troponin is a complex of three regulatory proteins (troponin C, troponin I, and troponin T) that is integral to muscle contraction in cardiac muscle. Troponin is an intracellular protein complex that is not normally present in the blood serum.

Troponin I is specific to cardiomyocytes and is useful as a diagnostic marker or therapeutic target for various heart disorders in particular as a highly specific marker for myocardial infarction or heart muscle cell death.

Upon cardiomyocyte damage troponin I is released into the circulation with the consequence of loss of contractile function.

Abnormally high troponin I levels in the blood are indicative of cardiomyocyte damage in a broad variety of cardiac diseases. These include instable angina pectoris [Bonaca et al., 2013], heart infarction [Mueller, 2013], myocarditis [Elamm et al., 2012], acute rejection of heart transplants [Labarrere et al., 2000], myocardial infarctions resulting from stent implantation [Zimarino et al., 2011], as well as traumatic surgical procedures, such as coronary artery bypass grafting [Moon et al., 2012].

Troponin I blood levels exceeding diagnostic cutoff representing the 99th percentile of a reference population are highly indicative for myocardial infarctions [Thomas et al, 2013].

The pathophysiological processes leading to cardiomyocyte damage are seen in insufficient blood supply through coronary arteries and/or concomitant cardiac inflammation.

Although medical therapy aimed at restoration of insufficient blood flow, mainly by clot lysis, stent implantation and coronary artery bypass surgery, are powerful treatments to prevent further cardiomyocyte damage in case of insufficient blood supply, the consequences of cardiac inflammation promoting cardiomyocyte damage are still a source of high disease burden.

Attempts have been made to suppress cardiac inflammation using anti-inflammatory strategies such as leukocyte depletion. These have not resulted in convincing therapies for lowering myocardial damage [Loberg et al., 2010]. Furthermore, troponin T is also broadly used and accepted as a marker for damage, both to the heart muscles as well as further muscles.

### Literature

Bonaca MP, Ruff CT, Kosowsky J, Conrad MJ, Murphy SA, Sabatine MS, Jarolim P, Morrow DA. Evaluation of the diagnostic performance of current and next-generation assays for cardiac troponin I in the BWH-TIMI ED Chest Pain Study. Eur Heart J Acute Cardiovasc Care. (2013) 2(3):195-202
Butler MW, Robertson I, Greene CM, O'Neill SJ, Taggart CC, McElvaney NG. elafin prevents lipopolysaccharide-induced AP-1 and NF-kappaB activation via an effect on the ubiquitin-proteasome pathway. J Biol Chem. (2006) 281(46):34730-5
Doucet A, Bouchard D, Janelle MF, Bellemare A, Gagné S, Tremblay GM, Bourbonnais Y. Characterization of human pre-elafin mutants: full antipeptidase activity is essential to preserve lung tissue integrity in experimental emphysema. Biochem J. (2007) 405(3):455-63
Elamm C, Fairweather D, Cooper LT. Pathogenesis and diagnosis of myocarditis. Heart. (2012) 98(11):835-40 Henriksen PA, Hitt M, Xing Z, Wang J, Haslett C, Riemersma RA, Webb DJ, Kotelevtsev YV, Sallenave JM. Adenoviral gene delivery of elafin and secretory leukocyte protease inhibitor attenuates NF-kappa B-dependent inflammatory responses of human endothelial cells and macrophages to atherogenic stimuli. J Immunol. (2004)172(7):4535-44
Labarrere CA, Nelson DR, Cox CJ, Pitts D, Kirlin P, Halbrook H. Cardiac-specific troponin I levels and risk of coronary artery disease and graft failure following heart transplantation. JAMA. (2000) 284(4):457-64
Loberg AG, Stallard J, Dunning J, Dark J. Can leucocyte depletion reduce reperfusion injury following cardiopulmonary bypass? Interact Cardiovasc Thorac Surg. (2011) 12(2):232-7
Moon MH, Song H, Wang YP, Jo KH, Kim CK, Cho KD. Changes of cardiac troponin I and operative mortality of coronary artery bypass. Asian Cardiovasc Thorac Ann. (2014) 22(1):40-5.
Mueller C. Use of high-sensitivity troponin for the diagnosis of acute myocardial infarction. Coron Artery Dis. (2013) 24(8):710-2
Nickel NP, Wang L, Li GG, Spiekerkoetter E., Rabinovitch M. The elastase inhibitor elafin restores endothelial cell homeostasis In pulmonary arterial hypertension and attenuates vascular remodeling In the Sugen/hypoxia rat model. Am J Respir Crit Care Med (2013) 187:A1031
Thomas S, Kavsak P, Devereaux PJ. Cardiac Troponin. Clinical Considerations for High-Sensitivity Assays. Clinical Laboratory News (2013) 39(4):8-10
Vanek T, Jares M, Straka Z; MSM0021620817 Study Group. Aprotinin reduces troponin I levels in OPCABG. Ann Thorac Surg. (2006) 82(5):1950-1
Zimarino M, Cicchitti V, Genovesi E, Rotondo D, De Caterina R. Isolated troponin increase after percutaneous coronary interventions: does it have prognostic relevance? Atherosclerosis (2012) 221(2):297-302

### SUMMARY OF THE INVENTION

The present invention relates to the use of elafin for the treatment and/or prevention of diseases or disorders which are associated with an increase in troponin I and/or T as further defined in the claims. This rise in troponin I and/or T is not associated with any increase or decrease of elastase activity in said disease or disorder. Nevertheless, the elafin can be used for the treatment of these diseases, although it was commonly believed that elafin is useful only in the context of those diseases which are associated with an increase in elastase. The present invention in a preferred embodiment relates to the use of a composition, using elafin, for protecting the heart muscle or other muscles from damage induced by abnormal blood flow, surgery, an accident with muscle damage and/or inflammation, which may result from, for example, a heart infarction.

Elafin treatment has been shown by the present inventor to lower myocyte damage. This is particularly surprising as this effect has also been shown to be independent of elastase activity. It is even more surprising as the inventor could show that the action of elafin is independent of its well known anti-inflammatory properties which are based on inhibition of elastase and proteinase 3.

The present invention also relates to the use of elafin for the treatment and/or prevention of diseases or disorders which are associated with a rise in troponin levels (in particular troponin I or T, particularly preferred troponin I) above the 99% percentile of the normal range.

The rise of troponin I or T has been shown by the present inventors to occur independently from the occurrence of increased elastase activity in subjects with any one of the above disorders.

It is noteworthy that the rise of troponins, as described herein, in particular of cardiac troponin I, is frequently associated with an adverse prognosis. This can be seen not only in e.g. all types of primary myocardial infarctions but also in secondary myocardial infarctions which occur as a secondary condition following another disease like all those conditions which are associated with an imbalance between myocardial oxygen supply and/or demand, e.g. coronary endothelial dysfunction, coronary artery spasm, coronary embolism, tachy-/brady-arrhythmias, aneamia, respiratory failure, hypotension and hypertension (see e.g. the Third Universal Definition of Myocardial Infarction, Thygesen et al, Eur. Heart J. 2012)

Coronary artery bypass surgery is a suitable model to investigate the principle of cardiomyocyte protection, as it combines cardiomyocyte damage due to insufficient blood supply during surgery and the consequences of cardiac inflammation as a result of the surgical procedure and reperfusion.

The present inventor has uncovered that the already well known release of elastase during the surgical procedure can lead only to an increase of enzymatically inactive elastase-alpha-1-proteinase inhibitor complexes, but not to a rise in free enzymatically active elastase in the circulation.

Thus, in such situations, it would have been thought that elafin could not be used for a treatment and/or prevention. The present inventor however was successful in showing that quite clearly, elafin can mediate beneficiary effects via different pathways and can thus also be used for the treatment and/or prevention of such disorders or diseases which are not mediated by or dependent on a rise of active elastase in the circulation.

The determination of the activity of elastase is well known in the art. Nakajima described in 1979 (J. Biol. Chem. 1979, 254:4027 - 4032) a suitable substrate for an elastase-activity assay, namely MeO-Suc-Ala-ALa-Pro-Val-pNA (NA = p-nitroaniline). This substrate was successfully used in the determination of serum elastase activity, e.g. in rabbits (Yoshimura K, Nakagawa S, Koyama S, Kobayashi T, Homma T. Roles of neutrophil elastase and superoxide anion in leukotriene B4-induced lung injury in rabbit. J Appl Physiol (1985). 1994 Jan;76(1):91-6). The hydrolysis of the substrate by elastase can measured by a known spectrophotometric method whereby e.g 1 mM of substrate is incubated with 0.1 ml sample in 0.1M tris(hydroxymethyl)aminomethane·HCl buffer at pH 8.0 containing 0.5 M NaCl in a final volume of 1.0 ml at 25°C. After incubation at 37°C for 24 h, the increase in the absorbance at 405 nm can be obtained and one unit of elastase activity can be defined as the quantity of enzyme that liberated 1 µmol of p-nitroaniline in 24 h. The activity of elastase can be determined in human serum, as shown e.g. by Nagamatsu et al used the same method as described above.

Elastase has been used as a marker for disease, in particular heart disease and here in particular as a marker for disorders like SIRS and MODS occurring during or after heart surgery and it is thus well known to the person of skill in the art how to detect elastase activity and how to evaluate the results.

The present invention is further described by the accompanying examples and Figures, wherein:
Figure 1 shows an approximate 7-fold increase of circulating complexes of elastase and its physiological inhibitor alpha-1-protease inhibitor
Figure 2 shows that free active elastase did not rise in serum during and after coronary artery bypass surgery.
Figure 3 depicts that postoperative rises of the pro-inflammatory cytokine IL-6 were not affected by the treatment with elafin.
Figure 4 depicts that postoperative rises of the C-reactive protein were not affected by the treatment with elafin.
Figure 5 shows the time course of plasma troponin I after commencement of coronary artery bypass surgery in patients treated with elafin versus placebo (mean, SD).
Figure 6 depicts that free active elastase in serum does not correlate with plasma troponin I after coronary artery bypass surgery.

### DETAILED DESCRIPTION OF THE INVENTION

1. An elafin polypeptide comprising the sequence of SEQ ID NO:1 or homologues, fragments or derivatives thereof, wherein said derivatives are polypeptides of SEQ ID NO:1 which are glycosylated, PEGylated, biotinylated, cyclized and/or oxidized, wherein the homologues are defined as having a sequence homology of more than 60 % compared to the polypeptide shown in SEQ ID NO: 1, and wherein the fragments, derivatives and homologues are defined as referring to those fragments, derivatives or homologues of the polypeptide of SEQ ID NO:1 which inhibit a release of troponins,for use in the prevention and/or treatment of disorders or diseases for all disorders involving damage to a cardiac muscle, wherein these disorders or diseases are associated with cell damage and/or cell death leading to troponin I and/or troponin T release and/or with a rise in plasma troponin I and/or troponin T, and wherein the terminology *"associated with cell damage leading to troponin I (or T) release and*/*or with a rise in plasma Troponin I (or T)"* is defined to pertain to patients which have an elevated troponin (I or T) above 0.04 µg/mlwherein said rise in troponin I and/or troponin T is not associated with an increase in elastase activity.
2. The polypeptide for the use of item 1, wherein the disorders or diseases are additionally not associated with an increase in the serine protease proteinase 3.
3. The polypeptide for the use of any one of items 1-2, wherein the disorder or disease is selected from disorders or diseases involving muscle damage or cell death in muscles, namely
   (secondary) heart diseases or disorders:
      stable coronary artery disease,
      chronic heart failure,
      atrial fibrillation,
      heart infarction,
      myocarditis,
      angina pectoris,
      acute pulmonary embolism,
      pulmonary arterial hypertension,
      coronary artery bypass surgery,
      cardiovascular surgery,
      renal insufficiency,
      acute rejection in patients with heart transplant,
   Muscular diseases:
      dermatomyositis,
      polymyositis,
      inclusion body myositis,
      Duchenne muscular dystrophy,
      rhabdomyolysis
      muscle damage after surgery or an accident,
      pulmonary arterial hypertension.
4. The polypeptide for the use of any one of items 1 to 3, wherein the homologues are defined as having a sequence homology of more than 70%, more than 80%, more than 90%, more than 95%, and even more preferably more than 98% compared to the polypeptide shown in SEQ ID NO: 1.
5. The polypeptide for the use of any one of items 1-4, wherein the polypeptide is to be administered parenterally, e.g. intravenously, subcutaneously, by inhalation, intramuscularly or intraarterially, preferably intravenously or subcutaneously.
6. The polypeptide for the use of any one of items 1-5, wherein the polypeptide is for a preventive use and is applied as a coating to an implant and/or stent.
7. The polypeptide for the use of any one of items 1-6, wherein the polypeptide is to be administered before, during or shortly after surgery.

According to one preferred aspect of the present invention a use of reducing cardiomyocyte damage is provided, the method comprising administering to a subject in need thereof a therapeutically effective amount of elafin (e.g. 200 mg) and e.g. a pharmaceutically acceptable carrier.

In a preferred embodiment, the use comprises a further step wherein the troponin I or troponin T level is determined before administration of elafin and/or wherein an elastase activity is determined before the administration of elafin.

The present polypeptide, homologue, derivative or fragment thereof can be given as a bolus administration before, during or after surgery, without the necessity of repeated administration and will still prevent the outbreak of the described diseases. A "bolus" administration in the present context shall mean an administration, which is carried out only once or twice, preferably once, to achieve the desired effect as described above. In the context of an intravenous bolus, the administration should preferably have a duration of not more than approximately 60 min. (infusion), preferably not more than 30 min. However, in an alternative embodiment the infusion could be continued for up to 12 h, or up to 24 h.

In specific embodiments, like stable coronary artery disease,
chronic heart failure, and (chronic) pulmonary arterial hypertension, it can be necessary to continue the elafin treatment beyond the above indicated time frames. The actual administration time and dosage regime will be determined by the practitioner and will depend on the remaining muscle damage and/or rise in troponin I and/or T levels.

In view of the fact that the present polypeptide, homologue, derivative or fragment thereof is particularly useful for the preparation of a pharmaceutical composition for the prevention and/or treatment of disorders and/or diseases not associated with an increase of elastase activity. The present invention contemplates in a further embodiment the use of the above-described compound for the preparation of wound dressings and/or as an additive to an organ perfusion medium.

In a preferred embodiment, the elafin compound as described above is administered intravenously. In another embodiment, the elafin compound can be administered subcutaneously.

The typical dosages of elafin are well known to the person of skill in the art and can be determined by the practitioner.

In a further preferred embodiment, administration can be carried out as a preventive administration e.g. before, during or shortly after surgery. In that case, a preferred administration is before surgery, even more preferred as a bolus (preferably once or twice and more preferred once) intravenously or subcutaneously.

"Before" surgery, in accordance with the present invention means an administration, which is carried out within 10 hours, preferably 6 hours, even more preferred, 4 hours, further preferred, 2 hours, or most preferred, within 1 min. to one hour before the beginning of surgery, or with the beginning of surgery.

"Shortly after" surgery in the present context means a period until no troponin I and/or T release is observable. This can be a period of up to several weeks, e.g. up to 4 weeks, or up to two weeks or of up to up to 1 week after the end of surgery; preferably within 3 days, more preferred, within 2 days and even more preferred within 6 hours after surgery.

Thus, for the first time, the present invention provides a possibility to prevent an outbreak or ameliorate the severity of the above-mentioned diseases. In particular after surgical intervention or after an accident involving damage to muscle cells, elafin can be used to prevent or treat disorders or diseases associated with muscle damage or muscle cell death, as indicated by a release of troponin I or troponin T or both, and without an observable elastase activity or an observable increase in elastase activity.

In accordance with the above described embodiments, the present elafin compound can also be used as an advantageous additive for the preparation of medical devices. One example of such a medical device would be an organ perfusion medium; similarly said elafin compound could be used for the preparation of a wound dressing, for the preparation of an additive to an organ perfusion medium, for the preparation of a medical sealant, for the preparation of a coating which is suitable for coating an implant or stent and for the implant or stent *per se.*

"Elafin Compound" or "elafin" as used hereinabove and hereinafter is interchangeable and always encompasses the polypeptide comprising the sequence of SEQ ID NO:1 as well as homologues, derivatives or fragments thereof, as defined in claim 1. Reference "Troponin" shall encompass in this application troponin T, troponin I, as well as the cardiac or skeletal subtypes thereof, if not explicitly mentioned otherwise.

An elafin compound will be further characterized in that it comprises the functionality to inhibit the release of troponin I and/or troponin T from muscle cells. This is measurable by testing for troponin levels in patients, as described below. An inhibition of troponin release will be assumed e.g. if the level of troponins is rised or is expected to rise above the 99^{th} percentile or above 0.04 µg/l and lowered by at least 20 %, preferably at least 30 % compared to a situation where no elafin is applied, either preventive or therapeutic. An inhibition of troponin release can e.g. also be determined to be present if the level of troponins stay below the 99^{th} percentile or below 0.04 µg/l in a condition where muscles have been damaged.

SEQ ID NO:1 is the following sequence: Ala Gln Glu Pro Val Lys Gly Pro Val Ser Thr Lys Pro Gly Ser Cys Pro Ile Ile Leu Ile Arg Cys Ala Met Leu Asn Pro Pro Asn Arg Cys Leu Lys Asp Thr Asp Cys Pro Gly Ile Lys Lys Cys Cys Glu Gly Ser Cys Gly Met Ala Cys Phe Val Pro Gln
Elafin was first isolated from the skin of patients with psoriasis, an inflammatory skin disease. It is a soluble protein with 57 amino acids and a molecular weight of about 6 kDa. Cloning of the elafin cDNA revealed that it is synthesized as a 12.3 kDa precursor (117 residues) which is processed intracellularly by cleavage of an N-terminal 22 residue signal sequence to give a 9.9 kDa protein called proelafin (or trappin-2, see below) which is secreted [Molhuizen HO, Alkemade HA, Zeeuwen PL, de Jongh GJ, Wieringa B, Schalkwijk J (1993) SKALP/elafin: an elastase inhibitor from cultured human keratinocytes. Purification, cDNA sequence, and evidence for transglutaminase cross-linking. J Biol Chem. 268(16):12028-32; Sallenave JM, Silva A (1993) Characterization and gene sequence of the precursor of elafin, an elastase-specific inhibitor in bronchial secretions. Am J Respir Cell Mol Biol. 8, 439-45; Schalkwijk J, Wiedow O, Hirose S (1999) The trappin gene family: proteins defined by an N-terminal transglutaminase substrate domain and a C-terminal four-disulphide core. Biochem J. 340, 569-77].

Up to now, the analysis of the primary structure of proelafin revealed the presence of two functional domains. The N-terminal domain (residues 1 - 60) contains four repeats of the sequence -Gly-Gln-Asp-X-Val-Lys- (SEQ ID NO:4) which is characteristic of transglutaminase substrates. The glutamine and lysine residues serve as acyl donors and acceptors, respectively, in the transglutaminase-mediated formation of isopeptide inter-protein cross-links [Molhuizen HO, Alkemade HA, Zeeuwen PL, de Jongh GJ, Wieringa B, Schalkwijk J (1993) SKALP/elafin: an elastase inhibitor from cultured human keratinocytes. Purification, cDNA sequence, and evidence for transglutaminase cross-linking. J Biol Chem. 268(16): 12028-32]. This portion of the molecule is often referred to as the cementoin domain. Tissue transglutaminase is able to cross-link proelafin to a variety of extracellular matrix proteins of the *stratum comeum* via this domain.

The second domain, consisting of the C-terminal 57 residues, harbours the protease inhibition function of proelafin and is identical to the 6 kDa soluble form of the molecule, i.e. elafin, originally isolated from psoriatic skin. This domain exhibits similarities to members of the whey acidic protein (abbreviated WAP) family in terms of its sequence, protein folding and arrangement of four characteristic disulphide bridges [Tamechika I, Itakura M, Saruta Y, Furukawa M, Kato A, Tachibana S, Hirose S (1996) Accelerated evolution in inhibitor domains of porcine elafin family members. J Biol Chem. 271, 7012-8; Tsunemi M, Matsuura Y, Sakakibara S, Katsube Y (1996) Crystal structure of an elastase-specific inhibitor elafin complexed with porcine pancreatic elastase determined at 1.9 Å resolution. Biochemistry 35, 11570-6]. The combination of transglutaminase substrate and WAP domains was subsequently demonstrated in other proteins for which a generic name "the trappin family" was coined. In order to clarify its affiliation with this protein family, the 9.9 kDa proelafin was termed trappin-2. The covalent attachment of proelafin to extracellular matrix proteins has little effect on its ability to inhibit elastase and proteinase-3 [Guyot N, Zani ML, Maurel MC, Dallet-Choisy S, Moreau T (2005). Elafin and its precursor trappin-2 still inhibit neutrophil serine proteinases when they are covalently bound to extracellular matrix proteins by tissue transglutaminase, Biochemistry 44, 15610-8], suggesting that transglutamination is a means of immobilizing this protease inhibitor in an active form. The mechanism by which elafin is released from proelafin has not been unequivocally elucidated. Proelafin produced in culture by a type II pneumocyte cell line was processed to elafin only in the presence of serum, indicating that cleavage occurs extracellularly, possibly prior to immobilization by transglutaminase [Sallenave JM, Silva A (1993) Characterization and gene sequence of the precursor of elafin, an elastase-specific inhibitor in bronchial secretions. Am J Respir Cell Mol Biol. 8, 439-45]. Consistent with this, tryptase, a mast cell protease was able to selectively release elafin from soluble proelafin [Guyot N, Zani ML, Berger P, Dallet-Choisy S, Moreau T (2005) Proteolytic susceptibility of the serine protease inhibitor trappin-2 (pre-elafin): evidence for tryptase-mediated generation of elafin. Biol Chem. 386, 391-9]. However, tryptase was inactive with proelafin cross-linked to fibronectin [Guyot N, Zani ML, Maurel MC, Dallet-Choisy S, Moreau T (2005). Elafin and its precursor trappin-2 still inhibit neutrophil serine proteinases when they are covalently bound to extracellular matrix proteins by tissue transglutaminase. Biochemistry 44, 15610-8]. Nevertheless, the fact that acid extracts of psoriatic scales only yield elafin and no cementoin domain, suggests that *in vivo* elafin is cleaved from proelafin cross-linked to skin matrix proteins. Furthermore, analysis of proelafin breakdown products in the urine revealed only the presence of C-terminal sequences, again pointing to the release of elafin by processing of cross-linked proelafin *in vivo* [Streit V, Wiedow O, Bartels J, Christophers E (1995) Antiprotease activity in urine of patients with inflammatory skin disorders. J Invest Dermatol. 105, 562-6]. The enzymes catalyzing elafin detachment from immobilized proelafin still remain to be identified.

The biosynthesis of proelafin is regulated at the transcriptional level and is strongly enhanced in response to the presence of epithelial inflammatory diseases, such as lymphocytic alveolitis and psoriasis. Physical injury, infections, irritation and exposure to ultraviolet radiation also induce elafin expression in the skin. Accordingly, proinflammatory stimuli such as the cytokines IL-1β and TNFα induce the expression of proelafin and elafin in various cultured cells, including respiratory cells and keratinocytes.

The structure of elafin, methods for its preparation, and its use for treating several disorders have also been addressed in the prior art, in particular in European Patent EP 0 402 068, US Patents 5,464,822 and 6,245,739 as well as published US Patent Application 2002/0187535. These references also disclose the primary structure of human elafin as depicted in SEQ ID NO: 1 and its capability to inhibit leukocyte elastase, in particular human leukocyte elastase and porcine pancreatic elastase. Moreover, methods for the preparation of elafin are described in these references. Preparation of derivatives and variants of elafin is also described in EP 0 662 516 and US 5,734,014.

In spite of the wealth of literature on elafin and the well studied effects thereof on elastase, there has - up to now - been no evidence that elafin has a further activity, which is useful for the treatment of disorders and diseases as defined herein and which is directly related to muscle cell damage, which is independent of elastase activity and which is correlated with a release of troponin I or troponin T.

It has also already been shown that elafin can be administered intravenously to animals such as rats without any side effects.

The invention generally relates to novel uses of polypeptides comprising the sequence depicted in SEQ ID NO: 1 or homologue, derivatives, or fragments of the sequence depicted in SEQ ID NO: 1, for the treatment of medical conditions for which a use of elafin has not yet been contemplated, as defined above and below.

In particular, elafin can be used for those diseases and/or disorders where no elastase activity or increase in elastase activity is observable. In particular, "no observable elastase activity" or "no observable increase in elastase activity" shall be - for the purposes of this application - the same as "not associated with an increase in active elastase" or "not associated with a rise of active elastase in the circulation" and shall be defined in the context of this application as described above, i.e. based on the well known assay for a detection of elastase activity. Elastase activity in the patients' sera is in a preferred embodiment determined by the cleavage of the chromogenic substrate MeO-Suc-Ala-Ala-Pro-Val-pNA, as is well known to the person of skill in the art and is described above in more detail.

During coronary artery bypass surgery neutrophil activation takes place with concomitant release of leukocyte elastase. A certain proportion of elastase in human serum remains uninhibited and enzymatically active. Interestingly, the present inventor could show that there are a number of disorders and diseases which are not associated with elastase activity, contrary to the earlier expectations. Although elastase levels as such might rise in such circumstances (e.g. after heart surgery or myocard infarction) there are cases, where this elastase is not active. Such cases would - in the prior art - have constituted a patient group which definitely would not have been treated with elafin, which was known solely for its effect on elastase activity.

Myocardial injury leads to a release of troponins into the circulation. This occurs e.g. in ischemic heart diseases, and other diseases which are accompanied by myocardial injuries, such as stable coronary artery disease, chronic heart failure, acute pulmonary embolism, or chronic pulmonary arterial hypertension. Troponin T release is similarly seen in these diseases but also serves as a marker for muscle cell damage in general, i.e. in further parts of the body. Furthermore, the terminology *"wherein the disorders or diseases are associated or correlated with (cell damage leading* to) *troponin I (or T) release and*/*or with a rise in plasma Troponin I(or T)"* shall mean in the context of this application that subjects/patients are included which have an elevated troponin (I or T, preferably I, even more preferred cardiac (c) troponin I and/or cardiac (c) troponin T for all disorders involving damage to a cardiac muscle) level above the 99% percentile of the normal range. This is the well established definition of an "elevated troponin level" and is used throughout the medical community. See for example the Thygesen K., et al, European Heart Journal (2012), 33, 2551- 2567, titled "Third Universal Definition of Myocardial Infarction" or "How to use high-sensitivity cardiac troponins in acute cardiac care" by Thygesen K., et al, European Heart Journal (2012) 33, 2252 - 2257, in particular table 1 thereof, which introduces and reviews peer-reviewed analytical evaluation data on cardiac troponin assays approved for routine diagnostics, which are both included herein by reference in their entirety. In a preferred embodiment, cTroponin I is measured by the Abbot Architect assay for troponins. The unit of measure is ng/ml. The reference range is <0.05 µg/l. In this assay, the 99^{th} percentile in normal has been reported to be in the range of 0.012 to 0.04 µg/l. The 99 percentile cutoff of 0.04 µg/l was verified. Thus, all troponin I results that exceed 0.04 µg/l in this assay are flagged as abnormal.

Thus, in the most preferred embodiment, a disease or disorder according to this invention can be treated with elafin if it is associated with a cTroponin I level of more than 0.04 µg/ml in an approved troponin-I assay, e.g. the STAT Troponin-I on Architect I by Abbott.

The disorders and/or diseases to be treated or prevented in the context of the present invention are all characterized in that they are not associated with an increase of elastase, as defined above and are additionally associated with cell damage leading to an increase of troponin I (and/or T) in plasma.

Such diseases encompass:
heart diseases or disorders or diseases with secondary heart involvement:
   stable coronary artery disease,
   chronic heart failure,
   atrial fibrillation
   heart infarction
   myocarditis
   angina pectoris,
   acute pulmonary embolism,
   pulmonary arterial hypertension
   coronary artery bypass surgery
   cardiovascular surgery
   renal insufficiency
   acute rejection in patients with heart transplant,
Muscular diseases:
   dermatomyositis,
   polymyositis,
   inclusion body myositis,
   Duchenne muscular dystrophy,
   rhabdomyolysis,
   renal insufficiency,
   muscle damage after surgery or an accident,
   pulmonary arterial hypertension.

In a particularly preferred embodiment, the diseases are selected from the group consisting of:
muscle damage after surgery or an accident,
coronary artery bypass surgery,
stable coronary artery disease,
chronic heart failure,
atrial fibrillation
heart infarction,
myocarditis,
angina pectoris,
cardiovascular surgery,
acute rejection in patients with heart transplant, or
pulmonary arterial hypertension
whereby this list is exemplary only and shall not be construed to be exhaustive.

All of these diseases are characterized by an increase of the troponin I- and/or T-level.

Although some of the above disorders or diseases were already treated with elafin before the date of this invention, the practitioner would not have considered such a treatment if the levels of elastase activity were not increased. Thus, the present invention presents an entirely new patient group which can now be treated with elafin. This treatment regime is actually entirely counter-intuitive to the earlier treatment regimes for elafin as it targets exactly those patients which would have been excluded based on the known earlier functionality of elafin.

As used herein, the term "homologue" refers to peptides or polypeptides which share a substantial degree of homology on the amino acid level with the sequence of SEQ ID NO: 1 over a certain stretch of its primary structure. In particular, the term "homologue" relates to polypeptides having a sequence (or comprising such sequence) which differs from the sequence depicted in SEQ ID NO: 1 by the substitution (or deletion) of one or more single amino acids. Generally, any amino acid from the sequence depicted in SEQ ID NO: 1 can be deleted or substituted against another amino acid as long as the inhibitory activity to the troponin rises of the polypeptide is not lost. Further polypeptides are also included which differ from the sequence of SEQ ID NO: 1 by the insertion of one or more additional amino acids. "One or more" in the above context always refers to 1-50, preferably 1-20, even more preferably 1-10, most preferably 1-5.

Based on the amount of identical amino acids, the sequence homology of a homologue according to the invention is more than 60%, preferably more than 70%, more than 80%, more than 90%, more than 95%, and even more preferably more than 98% compared to the polypeptide shown in SEQ ID NO: 1. The degree of amino acid homology may be evaluated by use of suitable computer programs known in the art, such as the GCG program package. A degree of homology, which is used throughout this description interchangeably with "identity", can be determined also by hybridization techniques, which are well known to a person skilled in the art. The above percentages of identity are thus determined in a preferred embodiment under stringent hybridization conditions. Identity may be measured using sequence analysis software (e.g., ClustalW at PBIL (Pole Bioinformatique Lyonnais) http://npsa-pbil.ibcp.fr).

As is known in the art, a number of different programs can be used to identify whether a nucleic acid or amino acid sequence has identity or similarity to a known sequence. Sequence identity or similarity may be determined using standard techniques known in the art, including, but not limited to, the local sequence identity algorithm of Smith & Waterman, Adv. Appl. Math. 2, 482 (1981), by the sequence identity alignment algorithm of Needleman & Wunsch, J. Mol., Biol. 48,443 (1970), by the search for similarity method of Pearson & Lipman, Proc. Natl. Acad. Sci. USA 85, 2444 (1988), by computerized implementations of these algorithms (GAP, BESTFIT, FASTA, and TFASTA in the Wisconsin Genetics Software Package, Genetics computer Group, 575 Science Drive, Madison, WI), the Best Fit sequence program described by Devereux et al., Nucl. Acid Res. 12, 387-395 (1984), preferably using the default settings, or by inspection.

The present invention also provides the inventive polypeptides expressed recombinantly in a suitable host from a corresponding polynucleotide. The present invention particularly provides such polynucleotides, which hybridize under stringent conditions to corresponding polynucleotides. As herein used, the term "stringent conditions" means conditions which permit hybridization between polynucleotides sequences and the polynucleotide sequences of SEQ ID NO: 1 where there is at least about 60%, preferably more than 70%, more than 80%, more than 90%, more than 95%, and even more preferably more than 98% identity..

Suitably stringent conditions can be defined by, e. g., the concentrations of salt or formamide in the prehybridization and hybridization solutions, or by the hybridization temperature, and are well known in the art. In particular, stringency can be increased by reducing the concentration of salt, by increasing the concentration of formamide, and/or by raising the hybridization temperature.

For example, hybridization under high stringency conditions may employ about 50% formamide at about 37°C to 42°C, whereas hybridization under reduced stringency conditions might employ about 35% to 25% formamide at about 30°C to 35°C. One particular set of conditions for hybridization under high stringency conditions employs 42°C, 50% formamide, 5x SSPE, 0.3% SDS, and 200 pg/ml sheared and denatured salmon sperm DNA. For hybridization under reduced stringency, similar conditions as described above may be used in 35% formamide at a reduced temperature of 35°C. The temperature range corresponding to a particular level of stringency can be further narrowed by calculating the purine to pyrimidine ratio of the nucleic acid of interest and adjusting the temperature accordingly. Variations on the above ranges and conditions are well known in the art.

Thus, the term homologue also comprises polypeptides which are longer than the sequence of SEQ ID NO: 1 and therefore comprise more amino acids, insofar as a part of their amino acid sequence shares substantial homology with the polypeptide of SEQ ID NO: 1. A polypeptide comprising the sequence depicted in SEQ ID NO: 1 which can be used according to the invention is, for example, the preproelafin shown in SEQ ID NO: 2 (Met Arg Ala Ser Ser Phe Leu lie Val Val Val Phe Leu lie Ala Gly Thr Leu Val Leu Glu Ala Ala Val Thr Gly Val Pro Val Lys Gly Gin Asp Thr Val Lys Gly Arg Val Pro Phe Asn Gly Gin Asp Pro Val Lys Gly Gln Val Ser Val Lys Gly Gln Asp Lys Val Lys Ala Gln Glu Pro Val Lys Gly Pro Val Ser Thr Lys Pro Gly Ser Cys Pro Ile Ile Leu lie Arg Cys Ala Met Leu Asn Pro Pro Asn Arg Cys Leu Lys Asp Thr Asp Cys Pro Gly lie Lys Lys Cys Cys Glu Gly Ser Cys Gly Met Ala
Cys Phe Val Pro Gln (Moihuizen, H.O. et al., J. Biol. Chem. 268 (16), 12028-12032 (1993)). As can be seen from that sequence, preproelafin comprises an additional N-terminal extension on the N-terminus and is post-translationally cleaved to provide the mature form. Preproelafin (117 amino acids) is first cleaved in proelafin (95 amino acids) and the N-terminal signal peptide (22 amino acids). During the terminal differentiation of keratinocytes (formation of the horny layer), proelafin becomes crosslinked to cornified envelope proteins by epidermal transglutaminase. The mature elafin is apparently released from these cornified envelope proteins by a yet unknown mechanism and can be extracted from horny layers of human skin, particularly from scales of patients suffering from psoriasis.

According to the present invention, the term "fragment" refers to any biologically active portion of the polypeptide in SEQ ID NO: 1 having the desired enzymatic activity on the inhibition of a rise of troponin. A fragment of elafin can consist of an amino acid sequence differing from the amino acid sequence in SEQ ID NO: 1 by the deletion of one or more amino acids at the N-terminus and/or C-terminus. For example, a fragment according to the invention may lack amino acid residue (s) 1, 1-2, 1-3, 1-4, 1-5, 1-6, 1-10 or 1-20 at the N-terminus of the polypeptide. Similarly, it may lack the corresponding residues at the C-terminus. Also, the elafin fragment can consist of the N-terminus of elafin. Moreover, a fragment may differ from the amino acid sequence in SEQ ID NO: 1 by lacking amino acid residues at both the N-terminus and C-terminus. For example, a fragment may consist of amino acids 6-30 of the polypeptide shown in SEQ ID NO: 1. Further comprised by the invention is the use of homologues of the fragments of the polypeptide shown in SEQ ID NO: 1.

The term "derivatives" refers to peptides or polypeptides which differ from the polypeptide shown in SEQ ID NO: 1 or from the homologs and fragments derived therefrom by well known amino acid modifications, such as glycosylation, PEGylation, biotinytation, cyclization and/or oxidation.

All above described fragments, homologues and derivatives of the invention as compared to the polypeptide of SEQ ID NO:1 have the function of inhibiting a release of troponins as defined above.

The polypeptides of the invention can be obtained as described in the prior art (see, for example, EP 0 402 068) or can be prepared by recombinant expression of the coding sequence as described by Sallenave, J.M. and Silva, A (Am. J. Respir. Cell Mol. Biol. 8 (4), 439-445 (1993)). The coding sequence is also provided in SEQ ID NO: 3

The polypeptides, homologues, derivatives and fragments as defined herein may be used as obtained or purified in a known and appropriate manner and formulated into pharmaceutical compositions, for example by admixture with a pharmaceutically acceptable diluent or carrier. Administration may be by way of various routes known in the art. In particular, administration may be effected parenterally, for example intra-nasally, intravenously, rectally, pulmonary, and by way of injection such as by way of intramuscular or subcutaneous injection. The pharmaceutical compositions will be formulated according to the mode of administration to be employed. For example, when the composition is to be administered by inhalation via the oral route or also e.g. intra-nasally, the composition may be formulated as a powdered aerosol; and when the composition is to be administered by way of injection it may be formulated as a sterile solution or suspension.

Suitable diluents including aqueous solutions and additives, such as buffers and surfactants may be added. Pharmaceutical compositions of the present invention also include controlled release formulations. For example, the polypeptides of the present invention may be encapsulated in a biodegradable polymer, or may be dispersed in a matrix of such a polymer, so that the polypeptide is released as the degradation of the polymer matrix proceeds.

Suitable biodegradable polymers for use in sustained release formulations include polyesters which gradually become degraded by hydrolysis when placed in an aqueous, physiological environment. A particular pharmaceutical composition which provides for extended release of a polypeptide is described in European Patent No. 0058481. In this composition a polylactide is employed, and when placed in an aqueous physiological-type environment, the polypeptide is released from the composition in a continuous manner until essentially all of the polypeptide has been released. Such polymers therefore offer the advantage of a highly localised target area, thus minimizing dosage and any potential side effects. This is in particular desirable as it is possible to continuously administer the elafin to the damaged cell area after injury of a muscle. This would apply for example to stents which have been inserted in a surgical procedure whereby this procedure is always accompanied by a certain risk of cell damage. Coating the stent with elafin will enable a continuous release of the elafin and will ensure that disorders associated with cell damage can be treated in a suitable fashion. The same of course applies to all further devices used when treating muscle damage, like e.g. natural or synthetic vascular grafts, natural or synthetic heart valves, indwelling catheters for dialysis, dental and orthopedic implants, artificial joints, materials for osteosynthesis, probes of cardiac pacemakers, medical sealants like fibrin sealants or bone cement, or long-term perfusion etc or external wound dressings.

In the present application, reference to "elafin" shall always encompass "homologues, derivatives and fragments" as described above.

Elafin may be administered systemically or by use of microspheres incorporated e.g. into a medical device, e.g. into a stent or implant, or coated or part of a wound dressing, which release the elafin in a controlled manner. The elafin can also be used in the form of elafin-containing polymers that release elafin in a controlled manner. The high stability of elafin to ethylene oxide sterilization is a further important aspect for its therapeutic application in this context, especially if is to remain active as a slow release drug or as a component in a medical device coating. The biocompatibility of elafin has been proven.

### EXAMPLES

Despite the lack of suppression of postoperative inflammation, Elafin had a pronounced impact on the cardiovascular damage associated with coronary artery bypass grafting surgery. The cardiovascular damage was assessed by troponin I release from cardiomyocytes. The plasma level of troponin I, a regulatory protein of cardiomyocyte contraction, is a specific marker for myocardial damage. Patients treated with Elafin at the start of surgery showed a significant reduction of troponin I release 6 hours after commencement of surgery and an overall reduction of the troponin I release over 48 hours (area under the curve) of 20 - 40%.

### Example 1:

43 Patients undergoing on-pump coronary artery bypass grafting surgery received a placebo treatment consisting of 250 mL of normal saline by intravenous infusion that was started at first skin incision and completed at least 20 min before cardiopulmonary bypass commenced.

Blood was drawn from these patients before commencing surgery (0 hours) and 6 hours after commencement of surgery. Plasma levels of immunoreactive elastase were quantified with a human elastase ELISA kit (Hycult, Uden, Netherlands) according to the instructions of the manufacturer. The human neutrophil elastase ELISA has been developed for the quantitative measurement of free and bound natural human neutrophil elastase in plasma with a lower detection level of 0.4 ng/ml.

The human neutrophil Elastase ELISA is a solid-phase enzyme-linked immunosorbent assay based on the sandwich principle. Samples and standards were captured by a solid bound specific antibody. Captured human neutrophil elastase was detected by a biotinylated tracer antibody, a streptavidin-peroxidase conjugate binding to the biotinylated tracer antibody, and peroxidase catalyzed reaction with tetramethylbenzidine. The enzyme reaction was stopped by the addition of oxalic acid. The absorbance at 450 nm was measured with a spectrophotometer. A standard curve was obtained by plotting the absorbance versus the corresponding concentrations of the human neutrophil elastase standards. The human neutrophil elastase concentration of samples, which were run concurrently with the standards, were determined from the standard curve.

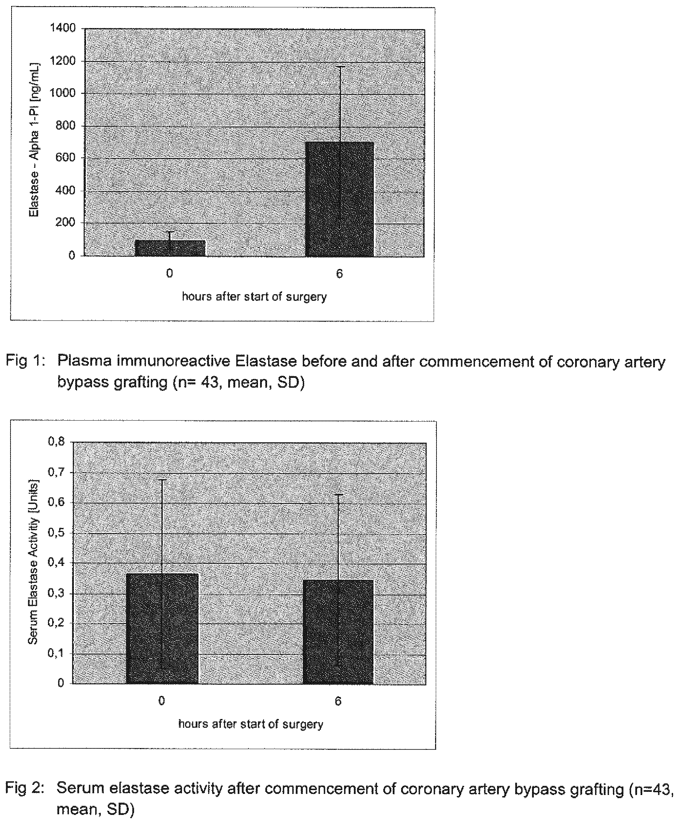

The results demonstrate immunoreactive human neutrophil elastase, representing free and alpha-1-protease inhibitor bound inactive elastase, increased significantly (approx. 7-fold) compared with preoperative levels. (Figure 1)

### Example 2:

43 Patients (the same as in example 1) undergoing on-pump coronary artery bypass grafting surgery received a placebo treatment consisting of 250 mL of normal saline by intravenous infusion that was started at first skin incision and completed at least 20 min before cardiopulmonary bypass commenced. Blood was drawn from these patients before commencing surgery (0 hours) and 6 hours after commencement of surgery. From the samples serum as prepared and the serum elastase activity was determined by the cleavage of the chromogenic substrate MeO-Suc-Ala-Ala-Pro-Val-pNA based on the method described by Nakajima, 1979, supra.

Human elastase hydrolyses the synthetic substrate MeO-Suc-Ala-Ala-Pro-Val-pNA and releases the yellow dye p-nitroaniline. The degree of p-nitroaniline release was quantified spectrophotometrically and used to determine the enzymatic activity of human neutrophil elastase. The neutrophil elastase activity Units represent delta OD 405 nm after 24 hour reaction at 37°C.

The results demonstrate that serum levels of enzymatically active neutrophil elastase do not increase during surgery (Figure 2). With the knowledge of increasing immunoreactive neutrophil elastase (example 1) it is concluded that the neutrophil elastase released is enzymatically inactive and may be completely bound to alpha1-proteinase inhibitor.

### Example 3:

44 Patients undergoing on-pump coronary artery bypass grafting surgery received a 200 mg dose of Elafin in 250 mL of normal saline by intravenous infusion that was started at first skin incision and completed at least 20 min before cardiopulmonary bypass commenced. A second group of 42 patients undergoing the same surgical procedure received 250 ml normal saline (placebo) per infusion instead.

Blood was drawn from patients and from the blood samples serum was prepared. Serum levels of interleukin 6 were determined by the use of a Human Interleukin 6 Immunoassay (R&D Systems Europe, Abingdon, UK) according to the instructions of the manufacturer. This assay employs the quantitative sandwich enzyme immunoassay technique. A monoclonal antibody specific for IL-6 has been pre-coated onto a microplate. Interleukin 6 standards and samples were pipetted into wells and any IL-6 present was bound by an immobilized antibody. After washing away any unbound substances, an enzyme-linked polyclonal antibody specific for IL-6 was added to the wells. Following a wash to remove any unbound antibody-enzyme reagent, a substrate solution was added to the wells. After an incubation period, an amplifier solution was added to the wells and color developed in proportion to the amount of IL-6 bound in the initial step. The color development was stopped and the intensity of the color is measured.

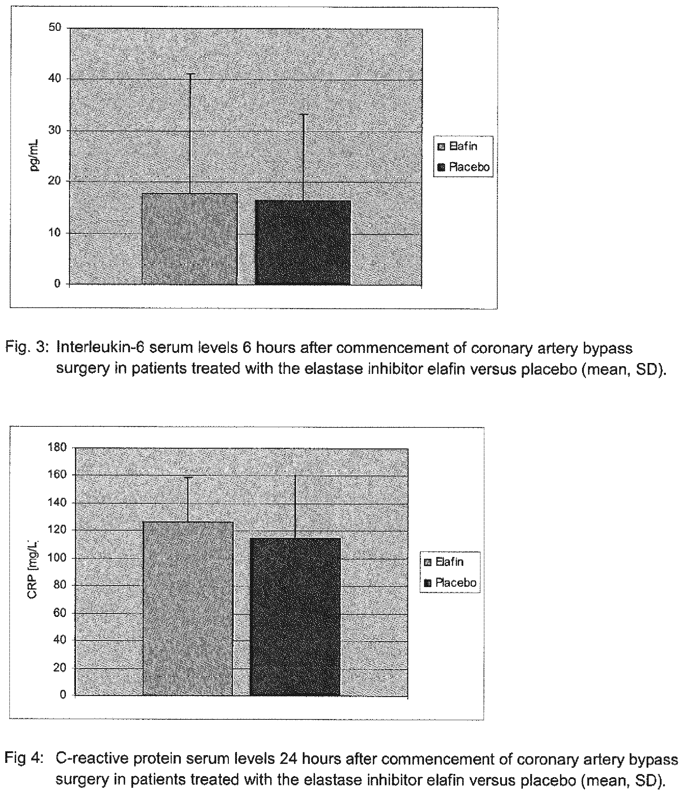

The results demonstrate that Elafin infusion had no effect on serum level of IL-6 as compared with a placebo treatment (Figure 3). This indicates that Elafin infusion had no influence on this marker of systemic inflammation.

### Example 4:

44 Patients undergoing on-pump coronary artery bypass grafting surgery received a 200 mg dose of Elafin in 250 mL of normal saline by intravenous infusion that was started at first skin incision and completed at least 20 min before cardiopulmonary bypass commenced. A second group of 42 patients undergoing the same surgical procedure received 250 ml normal saline (placebo) per infusion instead.

Blood was drawn from patients and from the blood samples serum was prepared. Serum levels of the inflammatory marker C-reactive protein (CRP) at time point 6 h were measured (Figure 4). The results demonstrate that Elafin infusion had no effect on serum level of CRP as compared with a placebo treatment. This indicates that Elafin infusion had no influence on this marker of systemic inflammation.

The perioperative treatment of patients with the elastase inhibitor Elafin consequently had no impact on the elastase-mediated postoperative inflammation when compared with placebo controls. Neither postoperative rises of the pro-inflammatory cytokine IL-6 (Fig. 3) nor of C-reactive protein (Fig. 4) were affected by the treatment with Elafin.

Despite the lack of suppression of postoperative inflammation, Elafin had a pronounced impact on the cardiovascular damage associated with coronary artery bypass grafting surgery (see Example 5 below).

### Example 5:

Patients undergoing coronary artery bypass grafting surgery were treated with either intravenous infusion of a 200 mg dose of Elafin in 250 mL of normal saline (Elafin group, n=44) or 250 mL of normal saline (placebo group, n=42) in a randomized, double-blinded clinical trial. The intravenous infusion was started at first skin incision and completed at least 20 min before cardiopulmonary bypass commenced.

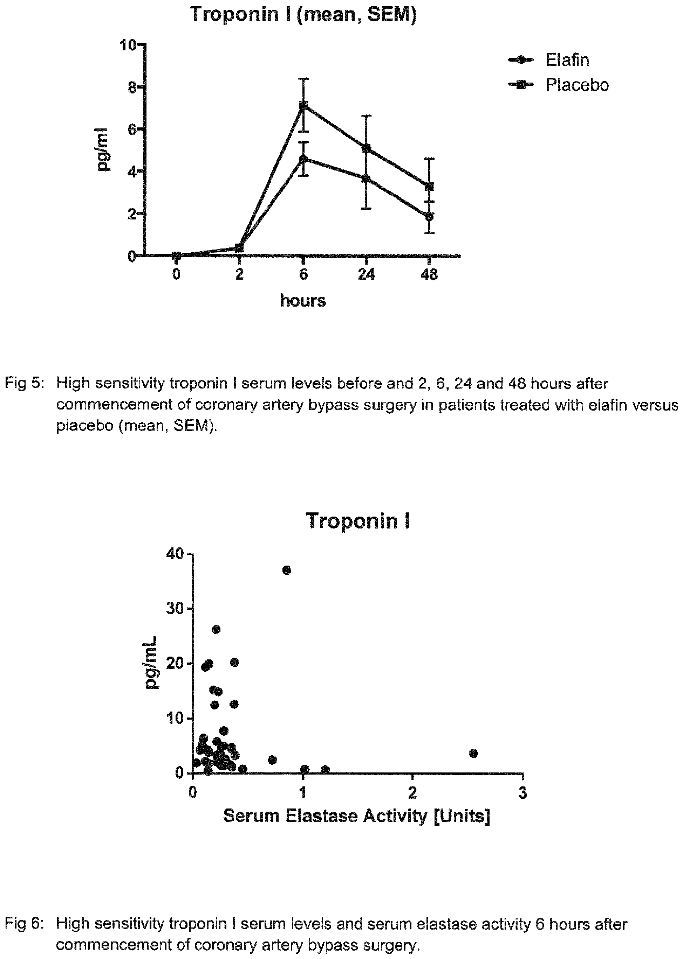

Blood samples were taken at baseline (time 0, skin incision) and at 2, 6, 24 and 48 h post-operatively. Plasma cTroponin I concentrations were quantified with the Abbott Architect highly-sensitive assay (Abbott Laboratories, ILL) according to the manufacturers instructions. This assay has a sensitivity of 0.01 ng/mL or less, with a 10% coefficient of variation of 0.032 ng/mL and a 99th percentile (male and female) of 0.012 ng/mL. cTroponin I is a regulatory protein of cardiomyocyte contraction and its presence in blood plasma is a specific indication of cardiomyocyte injury. Patients treated with Elafin at the start of surgery showed a significant reduction of cTroponin I release 6 hours after surgery and an overall reduction of the Troponin I release (area under the curve) over 48 hours (Figure 5). This clearly shows that Elafin lowers cardiomyocyte injury.

### Example 6:

In 42 Patients (from example 2) that were undergoing on-pump coronary artery bypass grafting surgery and received a placebo treatment consisting of 250 mL of normal saline elastase activity in the patients sera was determined by the cleavage of the chromogenic substrate MeO-Suc-Ala-Ala-Pro-Val-pNA (as in example 2) and plasma cTroponin I levels were determined (as in example 5).

The results demonstrate that serum levels of enzymatically active elastase do not correlate with cTroponin I (Pearson r correlation coefficient =0.02) indicating the myocardial damage leading to Tropinin I release is independent from elastase activity (Figure 6). This clearly shows that the mechanism that leading to cardiomycyte injury is independent from the serum elastase activity.

### Conclusion

Taken together the results show that while coronary artery bypass grafting leads to elevated elastase levels, essentially all of the released enzyme is sequestered by alpha-1-protease inhibitor (Figure 1), resulting in no increase in circulating elastase activity (Figure 2) and having no influence on markers of inflammation such as IL-6 (Figure 3) and CRP (Figure 4). The circulating elastase activity did not correlate with the troponin I release. Myocardial injury is expected to be a consequence of inflammation, partially driven by increased elastase activity. The observed prevention of myocardial injury, as assessed by Troponin I release, is obviously independent from the well known elastase inhibitory function of elafin.

The cardiovascular damage was therefore assessed by troponin I release from cardiomyocytes. The plasma level of troponin I, a regulatory protein of cardiomyocyte contraction, is a specific marker for myocardial damage. Patients treated with elafin at the start of surgery showed a significant reduction of troponin I release 6 hours after commencement of surgery and an overall reduction of the troponin I release over 48 hours (area under the curve) of 20 - 40%.

Thus, elafin treatment lowers cardiomyocyte damage. The mechanism is independent of its well known anti-inflammatory properties which are based on inhibition of elastase and proteinase 3.

## Claims

1. An elafin polypeptide comprising the sequence of SEQ ID NO:1 or homologues, fragments or derivatives thereof, wherein said derivatives are polypeptides of SEQ ID NO:1 which are glycosylated, PEGylated, biotinylated, cyclized and/or oxidized,
wherein the homologues are defined as having a sequence homology of more than 60 % compared to the polypeptide shown in SEQ ID NO: 1, and
wherein the fragments, derivatives and homologues are defined as referring to those fragments, derivatives or homologues of the polypeptide of SEQ ID NO:1 which inhibit a release of troponins,
for use in the prevention and/or treatment of disorders or diseases involving damage to a cardiac muscle, wherein these disorders or diseases are associated with cell damage and/or cell death leading to troponin I and/or troponin T release and/or with a rise in plasma troponin I and/or troponin T, and wherein the terminology *"associated with cell damage leading to troponin I (or T) release and*/*or with* a *rise in plasma Troponin I (or T)"* is defined to pertain to patients which have an elevated troponin (I or T) above 0.04 µg/l, wherein said rise in troponin I and/or troponin T is not associated with an increase in elastase activity.

2. The polypeptide for the use of claim 1, wherein the disorders or diseases are additionally not associated with an increase in the serine protease proteinase 3.

3. The polypeptide for the use of any one of claims 1 - 2, wherein the disorder or disease is selected from disorders or diseases involving muscle damage or cell death in muscles, namely
(secondary) heart diseases or disorders:
stable coronary artery disease,
chronic heart failure,
atrial fibrillation,
heart infarction,
myocarditis,
angina pectoris,
acute pulmonary embolism,
pulmonary arterial hypertension,
coronary artery bypass surgery,
cardiovascular surgery,
renal insufficiency,
acute rejection in patients with heart transplant,
Muscular diseases:
dermatomyositis,
polymyositis,
inclusion body myositis,
Duchenne muscular dystrophy,
rhabdomyolysis
muscle damage after surgery or an accident,
pulmonary arterial hypertension.

4. The polypeptide for the use of any one of claims 1 to 3, wherein the homologues are defined as having a sequence homology of more than 70%, more than 80%, more than 90%, more than 95%, and even more preferably more than 98% compared to the polypeptide shown in SEQ ID NO: 1.

5. The polypeptide for the use of any one of claims 1 -4, wherein the polypeptide is to be administered parenterally, e.g. intravenously, subcutaneously, by inhalation, intramuscularly or intraarterially, preferably intravenously or subcutaneously.

6. The polypeptide for the use of any one of claims 1 - 5, wherein the polypeptide is for a preventive use and is applied as a coating to an implant and/or stent.

7. The polypeptide for the use of any one of claims 1 - 6, wherein the polypeptide is to be administered before, during or shortly after surgery.

## Patentansprüche

1. Elafin-Polypeptid, umfassend die Sequenz von SEQ ID NO:1 oder Homologa, Fragmente oder Derivative davon, wobei die Derivative Polypeptide von SEQ ID NO:1 sind, welche glykosyliert, PEGyliert, biotinyliert, zyklisiert und/oder oxidiert sind,
wobei die Homologa definiert sind als eine Sequenzhomologie von mehr als 60 % aufweisend, verglichen dem in SEQ ID NO: 1 gezeigten Polypeptid, und wobei die Fragmente, Derivative und Homologa definiert sind als sich auf diese Fragmente, Derivative oder Homologa des Polypeptids von SEQ ID NO:1 beziehend, welche eine Ausschüttung von Troponinen inhibieren,
zur Verwendung bei der Prävention und/oder Behandlung von Störungen oder Krankheiten, welche Schädigung eines Herzmuskels involvieren, wobei diese Störungen oder Krankheiten mit Zellschädigung und/oder Zelltod assoziiert sind, welche(r) zu Troponin-I- und/oder Troponin-T-Ausschüttung führt/führen und/oder mit einem Anstieg an Plasma-Troponin-I und/oder -Troponin-T assoziiert sind, und wobei die Terminologie "mit *Zellschäden assoziiert, welche zu Troponin-I (oder -T)-Ausschüttung führen und*/*oder mit einem Anstieg an Plasma-Troponin-I (oder -T) assoziiert"* definiert ist als Patienten betreffend, welche ein erhöhtes Troponin (-I oder -T) über 0,04 µg/l haben,
wobei der Anstieg an Troponin-I und/oder Troponin-T nicht mit einer Steigerung an Elastase-Aktivität assoziiert ist.

2. Polypeptid zur Verwendung gemäß Anspruch 1, wobei die Störungen oder Krankheiten zusätzlich nicht mit an einer Steigerung der Serinprotease Proteinase-3 assoziiert sind.

3. Polypeptid zur Verwendung gemäß irgendeinem der Ansprüche 1 - 2, wobei die Störung oder Krankheit ausgewählt wird aus Störungen oder Krankheiten, welche Muskelschädigung oder Zelltod in Muskeln involvieren, nämlich
(sekundäre) Herzerkrankungen oder -störungen:
stabile koronär-arterielle Herzerkrankung,
chronische Herzinsuffizienz,
Vorhofflattern,
Herzinfarkt,
Myokarditis,
Angina Pectoris,
akute Lungenembolie,
pulmonal-arterielle Hypertonie,
koronar-arterielle Bypassoperation,
Kardiovaskular-Operation,
Niereninsuffizienz,
akute Abstoßungsreaktion in Patienten mit einem Herztransplantat,
Muskuläre Krankheiten:
Dermatomyositis,
Polymyositis,
Einschlusskörper-Myositis, Duchenne-Muskeldystrophie,
Rhabdomyolyse,
Muskelschädigung nach Operation oder einem Unfall,
pulmonal-arterielle Hypertonie.

4. Polypeptid zur Verwendung gemäß irgendeinem der Ansprüche 1 bis 3, wobei die Homologa definiert sind als eine Sequenzhomologie von mehr als 70%, mehr als 80%, mehr als 90%, mehr als 95%, und noch bevorzugter mehr als 98% aufweisend, verglichen mit dem in SEQ ID NO: 1 gezeigten Polypeptid.

5. Polypeptid zur Verwendung gemäß irgendeinem der Ansprüche 1 - 4, wobei das Polypeptid parenteral zu verabreichen ist, z.B. intravenös, subkutan, durch Inhalation, intramuskulär oder intraarteriell, bevorzugt intravenös oder subkutan.

6. Polypeptid zur Verwendung gemäß irgendeinem der Ansprüche 1 - 5, wobei das Polypeptid für eine präventive Verwendung ist und als eine Beschichtung auf ein Implantat und/oder Stent aufgetragen wird.

7. Polypeptid zur Verwendung gemäß irgendeinem der Ansprüche 1 - 6, wobei das Polypeptid vor, während oder kurz nach Operation zu verabreichen ist.

## Revendications

1. Polypeptide élafine comprenant la séquence SEQ ID NO:1 ou homologues, fragments ou dérivés de celui-ci, dans lequel lesdits dérivés sont des polypeptides de SEQ ID NO:1 qui sont glycosylés, PEGylés, biotinylés, cyclisés et/ou oxydés,
dans lequel les homologues sont définis comme présentant une homologie de séquence de plus de 60 % par rapport au polypeptide montré dans la SEQ ID NO:1, et
dans lequel les fragments, dérivés et homologues sont définis comme désignant les fragments, dérivés ou homologues du polypeptide de SEQ ID NO:1 qui inhibent une libération de troponines,
pour une utilisation dans la prévention et/ou le traitement de troubles ou de maladies impliquant une lésion d'un muscle cardiaque, dans lequel ces troubles ou ces maladies sont associés à des lésions cellulaires et/ou à une mort cellulaire entraînant la libération de troponine I et/ou de troponine T et/ou à une hausse de la troponine I et/ou de la troponine T plasmatique, et dans lequel l'expression « *associés à des lésions cellulaires entraînant la libération de troponine I (ou T) et*/*ou à une hausse de la troponine I (ou T) plasmatique* » est définie comme se rapportant à des patients qui ont un taux élevé de troponine (I ou T) supérieur à 0,04 µg/l,
dans lequel ladite hausse de la troponine I et/ou de la troponine T n'est pas associée à une augmentation d'activité de l'élastase.

2. Polypeptide pour l'utilisation selon la revendication 1, dans lequel les troubles ou les maladies ne sont pas non plus associés à une augmentation de la sérine protéase protéinase 3.

3. Polypeptide pour l'utilisation selon l'une quelconque des revendications 1-2, dans lequel le trouble ou la maladie sont choisis parmi des troubles ou des maladies impliquant des lésions musculaires ou une mort cellulaire dans des muscles, à savoir
maladies ou troubles cardiaques (secondaires) :
la coronaropathie stable,
l'insuffisance cardiaque chronique,
la fibrillation atriale,
l'infarctus du myocarde,
la myocardite,
l'angine de poitrine,
l'embolie pulmonaire aiguë,
l'hypertension artérielle pulmonaire,
le pontage coronarien,
la chirurgie cardiovasculaire,
l'insuffisance rénale,
le rejet aigu chez des patients ayant subi une transplantation cardiaque,
maladies musculaires :
la dermatomyosite,
la polymyosite,
la myosite à inclusions,
la myopathie de Duchenne,
la rhabdomyolyse,
une lésion musculaire après une intervention chirurgicale ou un accident,
l'hypertension artérielle pulmonaire.

4. Polypeptide pour l'utilisation selon l'une quelconque des revendications 1 à 3, dans lequel les homologues sont définis comme présentant une homologie de séquence de plus de 70 %, de plus de 80 %, de plus de 90 %, de plus de 95 % et encore plus préférentiellement de plus de 98 % par rapport au polypeptide montré dans la SEQ ID NO:1.

5. Polypeptide pour l'utilisation selon l'une quelconque des revendications 1-4, dans lequel le polypeptide doit être administré par voie parentérale, par exemple par voie intraveineuse, par voie sous-cutanée, par inhalation, par voie intramusculaire ou par voie intra-artérielle, de préférence par voie intraveineuse ou par voie sous-cutanée.

6. Polypeptide pour l'utilisation selon l'une quelconque des revendications 1-5, dans lequel le polypeptide est destiné à une utilisation préventive et est appliqué sous forme de revêtement sur un implant et/ou une endoprothèse.

7. Polypeptide pour l'utilisation selon l'une quelconque des revendications 1-6, dans lequel le polypeptide doit être administré avant, pendant ou rapidement après une intervention chirurgicale.
